# EUROPEAN PATENT SPECIFICATION

(11) **EP 1 680 106 B1**
(45) Date of publication and mention of the grant of the patent: **05.08.2009**
(21) Application number: 04791282.9
(22) Date of filing: 22.10.2004
(51) Int. Cl.: A61K 31/415, A61K 31/4155, A61K 31/4439, A61K 31/365, A61K 31/785, A61K 45/06, A61P 3/04

(54) **COMBINATION TREATMENT OF OBESITY INVOLVING 4,5-DIHYDRO-1H-PYRAZOLE DERIVATIVES HAVING CB1-ANTAGONISTIC ACTIVITY AND LIPASE INHIBITORS**
KOMBINATIONSBEHANDLUNG VON ADIPOSITAS MIT 4,5-DIHYDRO-1H-PYRAZOL-DERIVATEN MIT CB1-ANTAGONISTISCHER WIRKUNG UND LIPASE-HEMMERN
TRAITEMENT COMBINE DE L'OBESITE CONTENANT DES DERIVES DE 4,5-DIHYDRO-1H-PYRAZOLES AYANT UNE ACTIVITE ANTAGONISTE CB1 ET DES INHIBITEURS DE LIPASE

(30) Priority: 24.10.2003 EP 03103961
(43) Date of publication of application: 19.07.2006
(73) Proprietor: Solvay Pharmaceuticals GmbH, 30173 Hannover (DE)
(72) Inventor: ANTEL, Jochen, 31848 Bad Münder (DE); KRAUSE, Heinz Günter, 31303 Burgdorf (DE); GREGORY, Peter-Colin, 30559 Hannover (DE); WURL, Michael, 30826 Garbsen (DE); WALDECK, Harald, 30916 Isernhagen (DE); LANGE, Josephus Hubertus Maria, NL-1325 LP Almere (NL); KRUSE, Cornelis Gerrit, NL-3816 BJ Amersfoort (NL)
(74) Representative: Gosmann, Martin
(86) International application number: PCT/EP2004/052618
(87) International publication number: WO 2005/039566

(56) References cited:
- WO-A-01/70700
- WO-A-02/076949
- WO-A-03/026647
- WO-A-03/026648
- WO-A-03/051851
- WO-A-03/063781
- WO-A-03/064423
- WO-A-20/04012727
- WO-A-20/05000301
- WO-A1-20/05040130
- WO-A2-20/05039550
- US-A1- 2004 122 033
- HALFORD J C G ET AL: "Pharmacology of appetite suppression" PROGRESS IN DRUG RESEARCH 2000 SWITZERLAND, vol. 54, 2000, pages 25-58, XP001009413 ISSN: 0071-786X
- DOVE A: "Biotech weighs up the options in obesity" NATURE BIOTECHNOLOGY 2001 UNITED STATES, vol. 19, no. 1, 2001, pages 25-28, XP001176697 ISSN: 1087-0156
- M.H. BEERS AND R. BERKOW: "The Merck Manual of Diagnosis and Therapy, Seventeenth Edition" 1999, MERCK RESEARCH LABORATORIES , WHITEHOUSE STATION, N.J. , XP002271459 page 2255, column 2 - page 2258
- LANGE, JOS H. M. ET AL: "Synthesis, Biological Properties, and Molecular Modeling Investigations o Novel 3,4-Diarylpyrazolines as Potent and Selective CB1 Cannabinoid Receptor Antagonists" JOURNAL OF MEDICINAL CHEMISTRY (2004), 47(3), 627-643, 30 December 2003 (2003-12-30), XP001188902
- THEARLE M ET AL: "Obesity and pharmacologic therapy" ENDOCRINOLOGY AND METABOLISM CLINICS OF NORTH AMERICA 2003 UNITED STATES, vol. 32, no. 4, 2003, pages 1005-1024, XP008028335 ISSN: 0889-8529
- MOLNÁR D.: 'New drug policy in childhood obesity' INT. J. OBES. vol. 29, 2005, pages S62 - S65
- LANGGUTH P. ET AL: 'Biopharmazie', 2004, WILEY-VCH VERLAG GMBH & CO. KGAA
- http://www.dge.de/modules.php?name=News&fi le=print&sid=680
- BERKOWITZ R. ET AL: 'Behavior therapy and sibutramine for the treatment of adolescent obesity: a randomised controlled trial.' JAMA vol. 289, no. 14, 09 April 2003, pages 1805 - 1812
- NEED ET AL: 'The relationship of in vivo central CB1-receptor occupancy to changes in cortical monoamine release and feeding elicited by CB1-receptor antagonists in rats' PSYCHOPHARMACOLOGY vol. 184, 2006, pages 26 - 35

## Description

The present invention relates to a novel therapeutic and/or prophylactic treatment of obesity by administering a combination of a 4,5-dihydro-1H-pyrazole derivative having CB₁-antagonistic activity together with a further active principle, and to pharmaceutical products containing at least one of these CB₁-antagonistic compounds in combination with said further active principle for the treatment and/or prophylaxis of obesity as specified in the claims. The combination provided by the present invention of a 4,5-dihydro-1H-pyrazole showing potent Cannabis-1 (CB₁) receptor antagonistic activity with said further active principle are of particular utility for treating obesity, is juvenile patients and/or drug induced obesity in juvenile as well as adolescent patients.

Cannabinoids are present in the Indian hemp *Cannabis Sativa L.* and have been used as medicinal agents for centuries (Mechoulam, R.; Feigenbaum, J.J. Prog. Med. Chem. 1987, 24, 159). However, only within the past ten years the research in the cannabinoid area has revealed pivotal information on cannabinoid receptors and their (endogenous) agonists and antagonists. The discovery and the subsequent cloning of two different subtypes of Cannabinoid receptors (CB₁ and CB₂) stimulated the search for novel cannabinoid receptor antagonists (Munro, S.; Thomas, K.L.; Abu-Shaar, M. Nature 1993, 365, 61. Matsuda, L.A.; Bonner, T.I. Cannabinoid Receptors, Pertwee, R.G. Ed. 1995, 117, Academic Press, London). In addition, pharmaceutical companies became interested in the development of cannabinoid drugs for the treatment of diseases connected with disorders of the cannabinoid system. The wide distribution of CB₁ receptors in the brain, in combination with the strictly peripheral localization of the CB₂ receptor, makes the CB₁ receptor a very interesting molecular target for CNS-directed drug discovery in several areas of medical indications, e.g. psychiatric and neurological disorders are described in the state of the art as being of interest (Consroe, P. Neurobiology of Disease 1998, 5, 534. Pop, E. Curr. Opin. In CPNS Investigational Drugs 1999, 1, 587. Greenberg, D.A. Drug News Perspect. 1999, 12, 458).

From the international patent application WO 01/70700 4,5-dihydro-1H-pyrazole compounds are known which exhibit potent and selective cannabis CB₁-receptor antagonistic activity. These compounds have the formula (I) defined below, and have been suggested for use in the treatment of psychiatric disorders such as psychosis, anxiety, depression, attention deficits, memory disorders and appetite disorders, obesity, neurological disorders such as dementia, distonia, Parkinson's disease, Alzheimer's disease, epilepsy, Huntington's disease, Tourette's syndrome, cerebral ischemia, as well as for the treatment of pain disorders and other CNS-diseases involving cannabinoid neurotransmission, and in the treatment of gastrointestinal disorders and cardiovascular disorders.

It has now surprisingly been found that CB₁ antagonistic compounds (CB₁ antagonists), in particular 4,5-dihydro-1H-pyrazole derivatives which are potent and selective antagonists of the cannabis CB₁-receptor, as well as tautomers and salts thereof, due to their unique pharmacological profile are particularly suited in combination with at least one lipase inhibiting compound (lipase inhibitor) for the use in the manufacture of a medicament for the treatment and/or prophylaxis of obesity in juvenile patients and/or drug induced obesity in juvenile as well as adolescent patients. In this regard combinations of at least one CB1 antagonistic compound with at least one lipase inhibiting compound are highly valuable in providing medicaments for the treatment and/or prophylaxis of obesity in juvenile patients of any age, and particularly also in paediatric or juvenile obesity, and also in drug induced obesity in adolescent and juvenile patients.

Therefore, the invention pertains to the combination of a 4,5-dihydro-1H-pyrazole derivative of formula (I) which is a potent and selective antagonist of the cannabis CB₁-receptor, or a tautomer or salt thereof, with at least one lipase inhibiting compound, as specified in the claims.

In particular the present invention is based on the surprising finding that the 4,5-dihydro-1 H-pyrazole derivatives of the formula (I) which are potent and selective antagonists of the cannabis CB₁-receptor, tautomers thereof and salts thereof 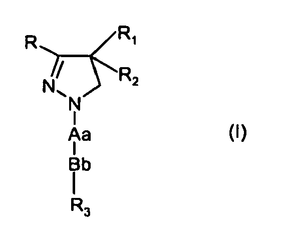 wherein
- R and R₁ are the same or different and represent phenyl, thienyl or pyridyl which groups may be substituted with 1, 2 or 3 substituents Y, which can be the same or different, from the group C₁₋₃-alkyl or alkoxy, hydroxy, halogen, trifluoromethyl, trifluoromethylthio, trifluoromethoxy, nitro, amino, mono- or dialkyl (C₁₋₂)-amino, mono- or dialkyl (C₁₋₂)-amido, (C₁₋₃)-alkyl sulfonyl, dimethylsulfamido, C₁₋₃-alkoxycarbonyl, carboxyl, trifluoromethylsulfonyl, cyano, carbamoyl, sulfamoyl and acetyl, or R and/or R₁ represent naphthyl,
- R₂ represents hydrogen, hydroxy, C₁₋₃-alkoxy, acetyloxy or propionyloxy,
- Aa represents one of the groups (i), (ii), (iii), (iv) or (v) 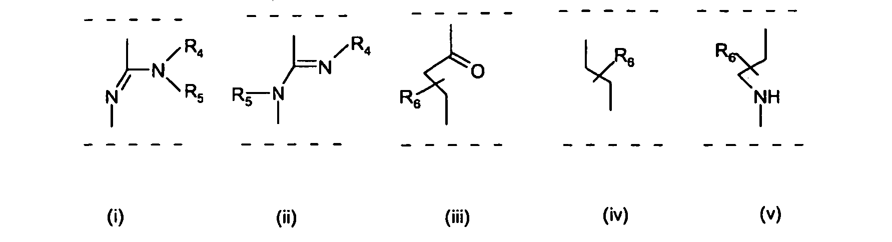 wherein
   - R₄ and R₅ independently of each other represent hydrogen or C₁₋₈ branched or unbranched alkyl or C₃₋₈ cycloalkyl or R₄ represents acetamido or dimethylamino or 2,2,2-trifluoroethyl or phenyl or pyridyl with the proviso that R₅ represents hydrogen,
   - R₆ represents hydrogen or C₁₋₃ unbranched alkyl;
- Bb represents sulfonyl or carbonyl,
- R₃ represents benzyl, phenyl, thienyl or pyridyl which may be substituted with 1, 2 or 3 substituents Y, which can be the same or different, or R₃ represents C₁₋₈branched or unbranched alkyl or C₃₋₈cycloalkyl, or R₃ represents naphthyl which due to their unique pharmacological profile are particularly suited in combination with at least one lipase inhibiting compound as specified in the claims for the use in the manufacture of a medicament for the treatment and/or prophylaxis of obesity in juvenile patients of any age, and particularly also for the treatment and/or prophylaxis of obesity in juvenile patients and/or drug induced obesity in juvenile as well as adolescent patients. In this regard combinations of the compounds of formula (I) together with lipase inhibiting compounds as specified in the claims are highly valuable in providing medicaments for the treatment and/or prophylaxis of obesity in juvenile patients of any age, and particularly also in pediatric or juvenile obesity, and in drug induced obesity, in juvenile as well as adolescent patients.

The outstanding unique pharmacological profile of CB₁ antagonistic compounds (CB₁ antagonists), and in particular of the 4,5-dihydro-1H-pyrazole derivatives as defined in formula (I) which are potent and selective antagonists of the cannabis CB₁-receptor, as well as tautomers and salts thereof, includes particularly high safety and tolerability also in combination with other drugs, in particular in combination with lipase inhibiting compounds according to the present invention. Thus the CB₁ antagonistic compounds in combination with lipase inhibiting compounds are particularly suitable also in patient groups with enhanced need of safety and tolerability, in particular such as juvenile patients and/or patients subject to long term treatment, e.g. in drug induced obesity.

This safety and tolerability of CB₁ antagonistic compounds in combination with lipase inhibiting compounds is advantageous in the treatment and/or prophylaxis of obesity in those patient populations where a single treatment is not sufficiently effective and a combination treatment and/or prophylaxis involving different medical or metabolic mechanisms is desired or required for achieving and stabilizing a defined degree of weight loss.

Hence, combination of CB₁ antagonistic compounds in combination with lipase inhibiting compounds according to the present invention is expected to be advantageous in the treatment and/or prophylaxis of obesity in juvenile patients of any age, and particularly also in pediatric or juvenile obesity, and in drug induced obesity, in juvenile as well as adolescent patients.

The CB₁ receptor modulating activity of the compounds of the invention makes them particularly useful in the treatment of juvenile obesity and drug induced obesity in juvenile well as adolescent patients, when used in combination with lipase inhibitors as specified in the claims. Examples of lipase inhibiting compounds the synthetic lipase inhibitor orlistat, panclicins, lipase inhibitors isolated from micro organisms such as lipstatin (from *Streptomyces foxytricim),* ebelactone B (from *Streptomyces aburaviensis),* synthetic derivatives of these compounds, 2-oxy-4H-3,1-benzoxazin-4-one derivatives like ATL-962 and structurally related compounds, 2-amino-4H-3,1-benzoxazin-4-one derivatives, as well as extracts of plants known to possess lipase inhibitory activity, for instance extracts of *Alpinia officinarum* Hance or compounds isolated from such extracts like 3-methylethergalangin (from A. officinarum).

The lipase inhibiting compound may also be a lipase inhibiting polymer. These lipase inhibiting compounds and their manufacture are well known in the state of the art.

The affinity of the compounds of formula (I) for cannabinoid CB₁ receptors is described in the WO 01/70700, e.g. it was determined using membrane preparations of Chinese hamster ovary (CHO) cells in which the human cannabis CB₁ receptor is stably transfected in conjunction with [3H]CP-55,940 as radioligand. After incubation of a freshly prepared cell membrane preparation with the [3H]-ligand, with or without addition of compounds of the invention, separation of bound and free ligand was performed by filtration over glass fibre filters. Radioactivity on the filter was measured by liquid scintillation counting.

The cannabinoid CB₁ antagonistic activity of compounds of formula (I) is also described in the WO 01/70700, and was determined by functional studies using CHO cells in which human cannabinoid CB₁ receptors are stably expressed. Adenylyl cyclase was stimulated using forskolin and measured by quantifying the amount of accumulated cyclic AMP. Concomitant activation of CB₁ receptors by CB₁ receptor agonists (*e*.*g*. CP-55,940 or (R)-WIN-55,212-2) can attenuate the forskolin-induced accumulation of cAMP in a concentration-dependent manner. This CB₁ receptor -mediated response can be antagonized by CB₁ receptor antagonists such as the compounds used in the present invention.

At least one centre of chirality is present (at the C₄ position of the 4,5-dihydro-1H-pyrazole moiety) in the compounds of the formula (I). The invention relates both to the novel use of racemates, mixtures of diastereomers and the individual stereoisomers of the compounds having formula (I). The invention also relates both to the novel use of the E isomer, Z isomer and E/Z mixtures of compounds having formula (I) wherein Aa has the meaning (i) or (ii) as described herein above.

According to one embodiment of the invention compound having formula (I) are used, wherein R is the group 4-chlorophenyl, R₁ is phenyl, R₂ is hydrogen, Aa is the group (i) wherein R₄ is hydrogen and R₅ is methyl, Bb is sulfonyl, and R₃ represents 4-chlorophenyl, and salts thereof. The compound having formula (I) used according to the invention may be a levorotatory enantiomer.

The compounds of formula (I) used in the present invention can be obtained according to known methods. A suitable synthesis for the compounds used according to the present invention is described for compounds of formula (I) in the international patent application WO 01/70700. For example compounds having formula (III) *(vide infra),* wherein R₂ represents hydrogen can be also obtained according to methods known, for example: a) EP 0021506; b) DE 2529689.

Example compounds of formula (I) having been prepared according to WO 01/70700 and being investigated include the e.g. the following compounds:
1) 3-(4-Chlorophenyl)-4,5-dihydro-4-hydroxy-4-phenyl-1 H-pyrazole
2) 3-(4-Chlorophenyl)-4,5-dihydro-N-((4-fluorophenyl)sulfonyl)-4-phenyl-1H-pyrazole-1-carboxamidine
3) 4,5-Dihydro-N-((4-fluorophenyl)sulfonyl)-3-(4-methoxyphenyl)-4-(4-methoxy-phenyl)-1H-pyrazole-1-carboxamidine
4) 4,5-Dihydro-3-(4-methoxyphenyl)-4-(4-methoxyphenyl)-N-((4-methoxyphenyl)sulfonyl)-1H-pyrazole-1-carboxamidine
5) 3-(4-Chlorophenyl)-4,5-dihydro-4-phenyl-N-((2,4,6-trimethylphenyl)sulfonyl)-1H-pyrazole-1-carboxamidine
6) 3-(4-Chlorophenyl)-4,5-dihydro-N-((4-fluorophenyl)sulfonyl)-4-hydroxy-4-phenyl-1H-pyrazole-1-carboxamidine
7) 3-(4-Chlorophenyl)-4,5-dihydro-N-(1-naphtoyl)-4-phenyl-1H-pyrazole-1-carboxamidine
8) 3-(4-Chlorophenyl)-4,5-dihydro-4-phenyl-N-(2-pyridoyl)-1H-pyrazole-1-carboxamidine
9) N¹,N¹-Dimethyl-N²-((4-chlorophenyl)sulfonyl)-3-(4-chlorophenyl)-4,5-dihydro-4-phenyl-1 H-pyrazole-1-carboxamidine
10) N-Methyl-N'-((4-chlorophenyl)sulfonyl)-3-(4-chlorophenyl)-4,5-dihydro-4-(3-pyridyl)-1H-pyrazole-1-carboxamidine
11) N-Methyl-N'-((4-chlorophenyl)sulfonyl)-3-(4-chlorophenyl)-4,5-dihydro-4-(4-pyridyl)-1H-pyrazole-1-carboxamidine
12) N¹,N¹-Dimethyl-N²-((4-chlorophenyl)sulfonyl)-3-(4-chlorophenyl)-4,5-dihydro-4-hydroxy-4-phenyl-1H-pyrazole-1-carboxamidine
13) N-Ethyl-N'-((4-chlorophenyl)sulfonyl)-3-(4-chlorophenyl)-4,5-dihydro-4-hydroxy-4-phenyl-1H-pyrazole-1-carboxamidine
14) N-Methyl-N'-(3-(trifluoromethyl)benzoyl)-3-(4-chlorophenyl)-4,5-dihydro-4-phenyl-1H-pyrazole-1-carboxamidine
15) N-Methyl-N'-((4-chlorophenyl)sulfonyl)-3-(4-chlorophenyl)-4,5-dihydro-4-phenyl-1 H-pyrazole-1-carboxamidine
16) N-Methyl-N'-((3-chlorophenyl)sulfonyl)-3-(4-chlorophenyl)-4,5-dihydro-4-phenyl -1 H-pyrazole-1-carboxamidine
17) N-Methyl-N'-((4-chlorophenyl)sulfonyl)-3-(5-chloro-2-thienyl)-4,5-dihydro-4-phenyl-1 H-pyrazole-1-carboxamidine
18) N-Propyl-N'-((4-fluorophenyl)sulfonyl)-3-(4-chlorophenyl)-4,5-dihydro-4-phenyl-1 H-pyrazole-1-carboxamidine
19) N-(2-Propyl)-N'-((4-fluorophenyl)sulfonyl)-3-(4-chlorophenyl)-4,5-dihydro-4-phenyl-1H-pyrazole-1-carboxamidine
20) N-Methyl-N'-((2-propyl)sulfonyl)-3-(4-chlorophenyl)-4,5-dihydro-4-phenyl-1H-pyrazole-1-carboxamidine
21) N-(2-Propyl)-N'-((4-chlorophenyl)sulfonyl)-3-(4-pyridyl)-4,5-dihydro-4-phenyl-1H-pyrazole-1-carboxamidine
22) N¹-Ethyl-N¹-methyl-N²-((4-chlorophenyl)sulfonyl)-3-(4-chlorophenyl)-4,5-dihydro-4-phenyl-1H-pyrazole-1-carboxamidine
23) N¹-Ethyl-N¹-methyl-N²-((4-fluorophenyl)sulfonyl)-3-(4-chlorophenyl)-4,5-dihydro-4-phenyl-1H-pyrazole-1-carboxamidine
24) N¹,N¹-Dimethyl-N²-((4-(trifluoromethyl)phenyl)sulfonyl)-3-(4-chlorophenyl)-4,5-dihydro-4-phenyl-1H-pyrazole-1-carboxamidine
25) N¹,N¹-Dimethyl-N²-((3-methylphenyl)sulfonyl)-3-(4-chlorophenyl)-4,5-dihydro-4-phenyl-1H-pyrazole-1-carboxamidine
26) N¹,N¹-Dimethyl-N²-((3-methoxyphenyl)sulfonyl)-3-(4-chlorophenyl)-4,5-dihydro-4-phenyl-1 H-pyrazole-1-carboxamidine
27) N-Ethyl-N'-((4-chlorophenyl)sulfonyl)-3-(4-chlorophenyl)-4,5-dihydro-4-phenyl-1H-pyrazole-1-carboxamidine
28) N-Dimethylamino-N'-((4-chlorophenyl)sulfonyl)-3-(4-chlorophenyl)-4,5-dihydro-4-phenyl-1 H-pyrazole-1-carboxamidine
29) N-Methyl-N'-((4-(trifluoromethyl)phenyl)sulfonyl)-3-(4-chlorophenyl)-4,5-dihydro-4-phenyl-1 H-pyrazole-1-carboxamidine
30) N¹,N¹-Dimethyl-N²-((2-methylphenyl)sulfonyl)-3-(4-chlorophenyl)-4,5-dihydro-4-phenyl-1H-pyrazole-1-carboxamidine
31) N-Methyl-N'-((2,4-difluorophenyl)sulfonyl)-3-(4-chlorophenyl)-4,5-dihydro-4-phenyl-1H-pyrazole-1-carboxamidine
32) N-Acetamido-N'-((4-chlorophenyl)sulfonyl)-3-(4-chlorophenyl)-4,5-dihydro-4-phenyl-1 H-pyrazole-1-carboxamidine
33) N-(2,2,2-Trifluoroethyl)-N'-((4-chlorophenyl)sulfonyl)-3-(4-chlorophenyl)-4,5-dihydro-4-phenyl-1H-pyrazole-1-carboxamidine
34) N-(2-Pyridyl)-N'-((4-chlorophenyl)sulfonyl)-3-(4-chlorophenyl)-4,5-dihydro-4-phenyl -1 H-pyrazole-1-carboxamidine
35) N-(4-Pyridyl)-N'-((4-chlorophenyl)sulfonyl)-3-(4-chlorophenyl)-4,5-dihydro-4-phenyl -1H-pyrazole-1-carboxamidine
36) N-Phenyl-N'-((4-chlorophenyl)sulfonyl)-3-(4-chlorophenyl)-4,5-dihydro-4-phenyl -1 H-pyrazole-1-carboxamidine
37) 3-(4-Chlorophenyl)-1-[3-((4-chlorophenyl)sulfonyl)butanoyl]-4,5-dihydro-4-phenyl-1H-pyrazole
38) 3-(4-Chlorophenyl)-1-[3-(phenylsulfonyl)propanoyl]-4,5-dihydro-4-phenyl-1 H-pyrazole
39) 3-(4-Chlorophenyl)-1-[3-((4-chlorophenyl)sulfonyl)propanoyl]-4,5-dihydro-4-phenyl-1H-pyrazole
40) 3-(4-Chlorophenyl)-4,5-dihydro-4-phenyl-1-[2-((3-(trifluoromethyl)phenyl)-sulfonyl)ethyl]-1H-pyrazole
41) 3-(4-Chlorophenyl)-1-[2-(benzylsulfonyl)ethyl]-4,5-dihydro-4-phenyl-1 H-pyrazole
42) 3-(4-Chlorophenyl)-1-[2-((4-chlorophenyl)sulfonyl)ethyl]-4,5-dihydro-4-phenyl-1H-pyrazole
43) 3-(4-Chlorophenyl)-1-[2-((4-chlorophenyl)sulfonyl)ethyl]-4,5-dihydro-4-hydroxy-4-phenyl-1H-pyrazole
44) N-[2-(3-(4-Chlorophenyl)-4,5-dihydro-4-phenyl-1H-pyrazol-1-yl)ethyl]-3-(trifluoromethyl)benzenesulfonamide

Lipase inhibiting compounds used in the combinations according to the present invention are as defined in the claims. Lipases are key enzymes in the digestive system which break down tri- and diglycerides, which are too large to be absorbed by the small intestine into fatty acids which can be absorbed. Since lipases are responsible for the hydrolysis of fat, a consequence of their inhibition selected from is a reduction in fat hydrolysis and absorption. Therefore, inhibition of lipases results in a reduction in the absorption of fat. The lipase inhibiting compound is preferably the synthetic lipase inhibitor orlistat 2-oxy-4H-3,1-benzoxazin-4-one derivatives having formula II as defined below like ATL-962, lipase inhibitors isolated from micro organisms selected from lipstatin, and ebelactone. Most preferred are orlistat, panclicins, ATL-962 and lipstatin.

Orlistat (tetrahydrolipstatin) and lipstatin are described in the US-patent US 4,598,089 and its European equivalent EP 0 129 748 B1 in more detail. The compounds are 2-hexyl-3-hydroxy-hexadecanoic acid lactone derivatives with the chemical names (2S,3S,5S,7Z,10Z)-5-((S)-2-formamido-4-methylvaleryloxy)-2-hexyl-3-hydroxy-7,10-hexadecadienoic acid lactone (lipstatin) and (2S,3S,5S)-5-((S)-2-4-methylvaleryloxy)-2-hexyl-3-hydroxy-hexadecanoic acid lactone (tetrahydrolipstatin). The compounds are known to be inhibitors of pancreas lipase which can be used for the prevention of treatment of obesity and hyperlipaemia, for which purpose they can be formulated as medicaments or incorporated into industrially prepared foodstuffs. Inhibition of pancreas lipase prevents the hydrolysis of dietary fats to give absorbable free fatty acids and monoglycerides, so that the fats are excreted unchanged. IC50's for lipstatin and tetrahydrolipstatin for inhibition of hydrolysis of triolein by porcine pancrease lipase are 0.07 and 0.18 mcg/ml, respectively.

Furthermore, there are suitable lipase inhibitors which are structurally related to orlistat and/or lipstatin and which are known as panclicins. These panclicines are derived from orlistat and contain a 4-ring lactone (Mutoh M; Nakada N; Matsukima S; Ohshima S; Yoshinari K; Watanabe J Location: Kanagawa, Japan Issue Date: 19-JAN-1995 Journal: J.Antibiot., 47, No. 12, 1369-75, 1994). The biological data of these panclicins may be summarized as follows: Panclicins A, B, C, D and E, structural analogs of tetrahydrolipstatin (THL), dose-dependently inhibited hydrolysis of triolein of fatty acids by porcine pancreatic lipase, with IC50 values of 2.9, 2.6, 0.62, 0.66 and 0.89 microM, respectively. The inhibitory activity of panclicins A and B (alanine moiety in place of leucine in THL) was 2-3-fold weaker than that of THL; in contrast, the inhibitory activity of panclicins C, D and E (glycine moiety in place of leucine in THL) was 2-fold stronger than that of THL. Panclicins A, B, C, D and E also potently inhibited plasma lipases with IC50 values of 1.0, 1.2, 0.29, 0.25 and 0,15 microM, respectively. Panclicins A and B inhibited plasma lipases with the same potency as THL, while panclicins C, D and E had a 3-6-fold greater inhibitory activity than THL. Panclicins A, B, C, D and E inhibited bacterial and fungal lipases with profiles similar to those for porcine pancreatic lipase. Panclicins inhibited pancreatic lipase irreversibly, but less irreversibly than THL. Panclicins A, B, C, D and E irreversibly inhibit pancreatic lipase.

Ebelactone B is described in the US-patent US 4,358,602 and its German equivalent DE 3 109 335 C1. Ebelactone A and ebelactone B belong to a group of compounds that exhibit activity to enhance the cell mediated immune response in living animals and they also inhibit inflammations in living animals. Thus they may be used in the immunological treatment of tumours and for enhancing anti-tumour agents such as bleomycins. The compounds have anti-esterase activity and anti-formylmethionine aminopeptidase activity. Admininistration to mice of these compounds at a dosage of 0.781-50 mg/kg (i.p.) or 0.5 mg/kg (per os) enhances the development of DTH response and the compounds show a potentiating effect on cell-mediated immunity. Ebelactone B reduces carragheenin-induced swelling in mice.

In addition, in the US patent US 6,624,161 and its corresponding international patent application WO 00/040569 and WO 00/40247 further lipase inhibiting compounds are described which are also suitable in the context of the present invention for combination with CB1 antagonistic compounds described herein. These patent documents US 6,624,161 and WO 00/040569 describe a series of compounds which are 2-oxy-4H-3,1-benzoxazin-4-one derivatives, including ATL-962, and their use in obesity and obesity-related disorders, including type 2 diabetes. The 2-oxy-4H-3,1-benzoxazin-4-one derivatives have the formula (II) or are a pharmaceutically acceptable salt, ester, or amide thereof: 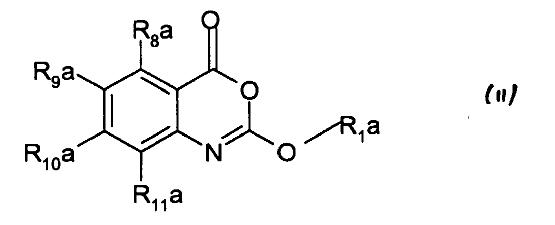 wherein:
R1a is
   (i) a C10-30 branched or unbranched alkyl , optionally substituted by one or more independently of C3-6 cycloalkyl, C3-6 cycloalkenyl, aryl, heteroaryl, reduced heteroaryl, --C(O)R13, --CO2R13, --SOR13, --SO2R13,-NR13R14, --OR13, --SR13, --C(O)NR13R14, --NR14C(O)R13, halogen, cyano, and nitro and/or optionally interrupted by one or more oxygen atoms with the proviso that any hetero atom in R1a must be separated from the exocyclic oxygen atom (or from any other heteroatom) by at least two carbon atoms;
   (ii) C2-25 alkenyl, C2-25 alkynyl, C3-6 cycloalkenyl, aryl-C2-25 alkenyl, heteroaryl-C2-25 alkenyl, reduced heteroaryl, reduced heteroaryl-C1-25 alkyl or a substituted derivative of any of the foregoing groups wherein the substituents are one or more independently of C1-6 alkyl, halosubstituted C1-6 alkyl, aryl, aryl-C1-6 alkyl, heteroaryl, reduced heteroaryl, reduced heteroaryl-C1-6 alkyl, C1-6 alkoxy, aryl-C1-6 alkoxy, --C(O)R13,-CO2R13, --SOR13, --SO2R13, --NR13R14, --OR13, --SR13, --C(O)NR13R14, --NR14C(O)R13, halogen, cyano, and nitro, with the proviso that any hetero atom in R1a must be separated from the exocyclic oxygen atom (or from any other heteroatom) by at least two carbon atoms;
(iii) a C2-9 alkyl group interrupted by one or more oxygen atoms and optionally substituted by one or more independently of C3-6 cycloalkyl, C3-6 cycloalkenyl, aryl, heteroaryl, reduced heteroaryl, -C(O)R13,-CO2R13, --SOR13, --SO2R13, NR13R14, OR13, SR13, --C(O)NR13R14, --NR14C(O)R13, halogen, cyano and nitro with the proviso that any hetero atom in R1a must be separated from the exocyclic oxygen atom (or from any other heteroatom) by at least two carbon atoms; or
(iv) a C1-9 alkyl group substituted by a group selected from --C(O)R13,-CO2R13, SOR13, SO2R13, NR13R14, OR13, SR13, C(O)NR13R14, NR14C(O)R13; tetrahydronaphthyl, pyridyl, pyrrolyl, piperidinyl, halogen, cyano, nitro, bicyclic aryl, bicyclic heteroaryl, monocyclic or bicyclic reduced heteroaryl, monocyclic heteroaryl other than imidazolyl;
(v) a phenyl group substituted by a group selected from OR17, --COR13,-CO2R13, SOR13, SO2R13, CONR13R14, NR14C(O)R13 ; halosubstituted C1-6 alkyl, aryl, arylC1-6 alkyl, heteroaryl and heteroarylC1-6 alkyl; or
(vi) a bicyclic aryl, bicyclic heteroaryl, monocyclic or bicyclic reduced heteroaryl, or monocyclic heteroaryl group other than imidazolyl, optionally substituted by a group selected from OR17, --COR13, --CO2R13, SOR13, SO2R13, CONR13R14, NR14 C(O)R13; halosubstituted C1-6 alkyl, aryl, arylC1-6 alkyl, heteroaryl and heteroarylC1-6 alkyl;
   where R13 and R14 each independently represents hydrogen, C1-10 alkyl, C2-10 alkenyl, C2-10 alkynyl, C3-6 cycloalkyl, C3-6 cycloalkenyl, aryl, arylC1-10·alkyl, heteroaryl, heteroarylC1-10 alkyl, reduced heteroaryl or reduced heteroaryl C1-10 alkyl, and R17 represents hydrogen or C2-10 alkenyl, C2-10 alkynyl, C3-6 cycloalkyl, C3-6 cycloalkenyl, aryl, arylC1-10 alkyl, heteroaryl, heteroarylC1-10 alkyl, reduced heteroaryl or reduced heteroarylC1-10 alkyl
and R8a, R9a, R10a and R11a
are each independently hydrogen, halo, hydroxy, amino, nitro, cyano, thiol, C1-10 alkyl,
C1-10 alkoxy, C1-10 cycloalkyl, C1-10 cycloalkoxy, C(O)R15, C(O)NR15R16, S(O)R15 or haloC1-10 alkyl;
where R15 and R16 each independently represent hydrogen or C1-10 alkyl with the proviso that when R8a, R9a, R10a, and R11a are H, R1a is not CH2CH2Cl or C3 alkenyl.
Furthermore in the international patent application WO 00/40247 related 2-amino-4H-3,1-benzoxazin-4-one derivatives are described as lipase inhibiting compounds for the treatment of obesity. In formula (II) then the -OR1a substituent is replaced by a -NR1R2 group with the definitions for R1 and R2 as given in the WO 00/40247.

The above group of structurally related compounds include ATL-962, an oral non-absorbed synthetic lipase inhibitor derived from Alizyme's pancreatic lipase inhibitor research program, is under development for the potential treatment of obesity and the potential management of type 2 diabetes. ATL-962 has the chemical name 2-hexadecyloxy-6-methyl-4H-3,1-benzoxazin-4-one. Preclinical studies showed that ATL-962 had similar efficacy to orlistat and no toxicity was observed. Clinical data for these compounds is also available in the public domain, e.g. resulting from clinical studies with ATL-962 in obesity.

Thus, the results from a phase Ib program with ATL-962 were presented at the International Congress of Obesity in Sao Paulo, Brazil. The three phase Ib trials involved a total of 99 healthy male volunteers in groups of seven or nine, given one of several doses of ATL-962 (66 subjects) or placebo (24 subjects), tid with food for 5 days. In one group the nine subjects were given orlistat (qv) 120 mg tid. Overall, ATL-962 was safe and well tolerated and showed evidence of efficacy as indicated by an increase in excretion of fat from the diet. Subjects given doses between 50 mg and 300 mg ATL-962 bid with meals excreted fat at an average of between 4.9 (+/-4.3) and 11.2 (+/-6.9) g/day compared to 1.4 (+/-1.0) g/day on placebo and 5.6 (+/-3.8) g/day on orlistat. Compared to placebo, 55% of subjects who received ATL-962 (50 mg to 300 mg) demonstrated a 3-fold or greater increase in fat excretion and 27% of subjects demonstrated a 7-fold or greater increase. There was evidence of dose-dependency. Adverse events and their frequency were similar between ATL-962 and placebo and were mainly gastrointestinal, with the predominant event being oily stool.

The results of a multicenter, randomized, double-blind, parallel-group trial (phase IIb study), involving 370 clinically obese patients, was being performed in specialist clinics in 5 European countries, and in September 2003 preliminary results were reported. All dose levels of ATL-962 (60, 120 and 240 mg) demonstrated a significant reduction in weight, compared to placebo, for all treatment groups. There was no difference in the extent of weight loss between treatment groups. LDL-cholesterol decreased in the treatment groups, but not for placebo. There was no difference in HDL-cholesterol levels in the treatment groups, whilst it increased in placebo-treated patients. Total cholesterol decreased in the treatment groups, whilst placebo showed an increase. ATL-962 was safe and generally well tolerated.

The lipase inhibiting compounds of formula (II) like ATL-962 and structurally related compounds are described in more detail in the US patent US 6,624,161 and its corresponding international patent application WO 00/040569, and can be obtained according to known methods. A suitable synthesis for the lipase inhibiting compounds of formula (II) is described also in the US 6,624,161 and international patent application WO 00/040569.

Pharmaceutically acceptable salts, hydrates and solvates of all the above described lipase inhibiting compounds may also be used in the context of the present invention.

The 4,5-dihydro-1H-pyrazole derivative which is a potent and selective antagonist of the cannabis CB₁-receptor of formula (I), or a tautomer or salt thereof, and the lipase inhibiting compound used according to the invention can be brought into forms suitable for treatment and/or prophylaxis of obesity, e.g. for juvenile or pediatric administration, as well as for the administration in treating drug induced obesity in juvenile as well as adolescent patients by means of usual processes using pharmaceutical excipients, auxiliary substances and/or liquid or solid carrier materials. As therapeutic agents, the CB₁ antagonistic compound and/or the lipase inhibiting compounds may be contained together with (conventional) pharmaceutical excipients, adjuvants and/or auxiliaries in pharmaceutical preparations such as tablets, capsules, suppositories or solutions. These pharmaceutical preparations may be prepared according to known methods, using conventional solid or liquid vehicles such as lactose, starch or talc, or liquid paraffins and/or using (conventional) pharmaceutical excipients, adjuvants and/or auxiliaries, such as tablet disintegrating agents, solubilisers or preservatives.

Hence, in a further aspect the invention also pertains to a pharmaceutical product containing at least one 4,5-dihydro-1 H-pyrazole derivative of formula (I) which is a potent and selective antagonist of the cannabis CB₁-receptor, or a tautomer or salt thereof, in combination with at least one lipase inhibiting compound as specified in the claims, for use in the treatment and/or prophylaxis of obesity is juvenile patients and/or for use is the treatment and/or prophylaxis of drug induced obesity in juvenile as well as adolescent patients. A preferred pharmaceutical product contains at least one compound of formula (I) as defined above in combination with at least one lipase inhibiting compound as specified in the claims as combined active components. Particular pharmaceutical products according to the invention, are characterized in that the at least one CB₁ antagonistic compound having formula (I) as defined above, or the tautomer or salt thereof, and the at least one lipase inhibiting compound as specified in the claims each are present in an amount effectively suited for the treatment and/or prophylaxis of obesity in a juvenile patient in need of such treating. In a further embodiment of the invention the CB₁ antagonistic compound of formula (I), and the lipase inhibiting compound as specified in the claims are each present in the pharmaceutical product in an amount effectively suited for the treatment and/or prophylaxis of drug induced obesity in juvenile as well as adolescent patients in need of such treating. In the pharmaceutical product according to the invention the CB₁ antagonistic compound having formula (I), or the tautomer or salt thereof, is used preferably in combination with at least one lipase inhibiting compound selected from the group of orlistat, panclicins, ATL-962 and lipstatin.

Also disclosed is a pharmaceutical product containing as a medicament a CB₁ antagonistic compound having formula (I) as defined in claim 1 or a tautomer or salt thereof, and a leaflet indicating that said CB₁ antagonistic compound may be administered in combination with a lipase inhibiting compound for simultaneous, separate or step-wise administration in the treatment and/or prophylaxis of obesity in juvenile patients and/or drug induced obesity in juveniles as well as in adolescent patients.

According to the invention the CB₁ antagonistic compound having formula (I) as defined above, or a tautomer or salt thereof, is to be administered in combination with the lipase inhibiting compound as defined in the claims by simultaneous, separate or step-wise administration route.

The compounds used in the combinations or products according to the present invention each are preferably administered to a patient in need thereof and in a quantity sufficient to prevent and/or treat the symptoms of the condition, disorder or disease. For all aspects of the invention, particularly medical ones, the administration of a compound or product has a dosage regime which will ultimately be determined by the attending physician and will take into consideration such factors such as the compound being used, animal type, age, weight, severity of symptoms, method of administration, adverse reactions and/or other contraindications. Specific defined dosage ranges can be determined by standard design clinical trials with patient progress and recovery being fully monitored. Such trials may use an escalating dose design using a low percentage of the maximum tolerated dose in animals as the starting dose in man.

The physiologically acceptable compounds used in the combinations or products according to the present invention each are will normally be administered in a daily dosage regimen (for an adult patient (reference)) of, for example, an oral dose of between 1 mg and 2000 mg, preferably between 30 mg and 1000 mg, e.g. between 10 and 250 mg or an intravenous, subcutaneous, or intramuscular dose of between 0.1 mg and 100 mg, preferably between 0.1 mg and 50 mg, e.g. between 1 and 25 mg of the compound of the formula (I) or a physiologically acceptable salt thereof calculated as the free base, the compound being administered 1 to 4 times per day. Suitably the compounds will be administered for a period of continuous therapy, for example for a week or more. For a juvenile patient usually a part of the oral dose for an adult patient is administered, e.g. 1 fifth to 1 half of the oral dose described before for an adult patient.

Preferably, in one embodiment of the invention the claimed use is directed to the treating of obesity in juvenile patients. In another preferred embodiment of the invention the claimed use is directed to the treating of drug induced obesity in juvenile or adolescent patients. This drug induced obesity may be in particular caused by drugs like atypical antipsychotics.

In one embodiment of the invention the claimed use is directed to the treating of obesity in juvenile patients. Thus, it is advantageous that Cannabinoid antagonists in combination with lipase inhibitors are particularly suitable for the treatment of Childhood Obesity and related Comorbidities as for example Type 2 Diabetes. There is a clear medical need for improved therapy as obesity has become an increasingly important medical problem not only in the adult population but increasingly in children and (young and older) adolescents. In national surveys from the 1960s to the 1990s in the United States, the prevalence of overweight in children grew from 5% to 11% (Sorof and Daniels 2002). In Canada as another example childhood obesity has tripled in the past 20 years (Spurgeon 2002). Obesity in childhood causes a wide range of serious complications, and increases the risk of premature illness and death later in life, raising public-health concerns (Ebbeling, Pawlak et al. 2002). Over the last decades a tremendous increase of cases of type 2 diabetes was observed, especially also in children. This epidemic trend is clearly reflecting the increasing rates of obesity. Type-2-diabetes was in the past considered a disease of adults and older individuals, not a pediatric condition (Arslanian 2002). One of the main risk factor of pediatric type 2 diabetes is obesity.

Type 2 diabetes in children (as is in adults) is part of the insulin resistance syndrome (Rosenbloom 2002) that includes hypertension, dyslipidemia and other atherosclerosis risk factors, and hyperandrogenism seen as premature adrenarche and polycystic ovary syndrome. Other outcomes related to childhood obesity include left ventricular hypertrophy, nonalcoholic steatohepatitis, obstructive sleep apnea, orthopedic problems, and severe psychosocial problems.

In addition primary hypertension has become increasingly common in children again associated obesity as a major independent risk factor. Obese children are at approximately a 3-fold higher risk for hypertension than non-obese children (Sorof and Daniels 2002). The benefits of weight loss for blood pressure reduction in children have been demonstrated in both observational and interventional studies.

Public concerns are rising because of a rapid development of the childhood obesity epidemic in genetically stable populations. Driving factors are assumed to be mainly adverse environmental factors for which straightforward recommendations of life style modifications exists. Obesity and it's related comorbidities are very serious medical conditions and state of the art measures and treatment of obesity and especially childhood obesity remain largely ineffective at the time being (Ebbeling, Pawlak et al. 2002). The management of type 2 diabetes is also especially difficult in children and the adolescent age group (Silink 2002). Craving for and over consumption of palatable food is one of the important factors of life-style related obesity in humans and especially also in children and adolescents. Treatment of type 2 diabetes and other co-morbid conditions by the degree of metabolic derangement and symptoms: The only data on the use of oral hypoglycemic agents in children with type 2 diabetes has been with metformin (Rosenbloom 2002).

Thus, CB₁ antagonists used according to the present invention in combination with lipase inhibitors offer a unique opportunity for the treatment of obesity by interacting with these "driving forces". They are superior to current medical treatments and especially suited for adolescent as well as pediatric treatment because of their outstanding safety profile and/or tolerability and surprisingly beneficial combination effects. Besides efficacy, the treatment of obesity, especially the treatment of childhood obesity, dictated by safety.

Obesity in childhood is a medical condition that is likely to require long-term management. The safety profile of CB₁ antagonists according to the present invention in combination with lipase inhibitors as specified is the claims are suggested to be superior to current standard medications, and these CB₁ antagonists in combination with lipase inhibitors as specified in the claims will be especially suited for the treatment and prevention of obesity in juveniles and in childhood obesity and related co-morbidities.

### Literature:

Arslanian, S. (2002). "Type 2 diabetes in children: clinical aspects and risk factors." Horm Res 57 Suppl 1: 19-28.
Ebbeling, C. B., D. B. Pawlak, et al. (2002). "Childhood obesity: public-health crisis, common sense cure." Lancet 360(9331): 473-82.
Rosenbloom, A. L. (2002). "Increasing incidence of type 2 diabetes in children and adolescents: treatment considerations." Paediatr Drugs 4(4): 209-21.
Silink, M. (2002). "Childhood diabetes: a global perspective." Horm Res 57 Suppl 1: 1-5.
Sorof, J. and S. Daniels (2002). "Obesity hypertension in children: a problem of epidemic proportions." Hypertension 40(4): 441-7.
Spurgeon, D. (2002). "Childhood obesity in Canada has tripled in past 20 years." Bmi 324(7351): 1416.

In another embodiment of the invention the claimed use is directed to the treating of drug induced obesity in juvenile or adolescent patients. Drug induced weight gain is also of major concern and subject to high medical need of improved treatments. Again, in this context the CB₁ antagonists in combination with lipase inhibitors according to the present invention are suggested to be superior to current standard medications, and these CB₁ antagonists in combination with lipase inhibitors will be especially suited for the treatment and prevention of drug induced obesity in juvenile as well as in adolescent patients.

Regarding drug induced weight gain, it is reported by Zimmermann, U., T. Kraus, et al. (2003, "Epidemiology, implications and mechanisms underlying drug-induced weight gain in psychiatric patients." J Psychiatr Res 37(3): 193-220) that body weight gain frequently occurs during drug treatment of psychiatric disorders and is often accompanied by increased appetite or food craving. While occurrence and time course of this side effect are difficult to predict, it ultimately results in obesity and the morbidity associated therewith in a substantial part of patients, often causing them to discontinue treatment even if it is effective. Weight gain appears to be most prominent in patients treated with some of the second generation antipsychotic drugs and with some mood stabilizers. Marked weight gain also frequently occurs during treatment with most tricyclic antidepressants.

Very large weight gains are associated with drugs like for example the atypical antipsychotics clozapine and olanzapine. Some atypical antipsychotics, however, tend to cause significant weight gain, which may lead to poor compliance and other adverse health effects (Nasrallah, H. (2003). "A review of the effect of atypical antipsychotics on weight." Psychoneuroendocrinology 28 Suppl 1: 83-96.). The mechanisms involved in antipsychotic drug-related weight gain are as yet uncertain, although serotoninergic, histaminic, and adrenergic affinities have been implicated along with other metabolic mechanisms. The atypical antipsychotics vary in their propensity to cause weight change with long-term treatment. Follow-up studies show that the largest weight gains are associated with clozapine and olanzapine, and the smallest with quetiapine and ziprasidone. Risperidone is associated with modest weight changes that are not dose related. Given the equivalent efficacy of atypical antipsychotics, weight-gain profile is a legitimate factor to consider when constructing an algorithm for treatment due to the serious medical consequences of obesity. In this regard co-administration of a CB₁ antagonist in combination with lipase inhibitors according to the invention is suggested to work beneficially.

### Experimental protocol for study of CB₁ antagonistic compound plus lipase inhibitor effects on obesity

The beneficial pharmacological effects of the combination of a CB₁ antagonist with a lipase inhibitor according to the invention can be shown by standard experimental animal models by measuring the influence of the administered combination of a CB₁ antagonist with a lipase inhibitor on the driving and characteristic parameters associated with obesity.

For investigation of the influence of the combination of a CB₁ antagonist with a lipase inhibitor on obesity the body weight gain in rats may be measured as a pharmacological indicator. Here fore, the following experimental protocol for rats may be applied:

The rats will have unlimited access to feed for two 2.5h periods per day, during the dark phase of a reversed 12h/12h light cycle, e.g. lights are put on at 21.15h and put off at 09.15h. The rat s will be offered a high fat, high sucrose diet (Western diet). The lipase inhibitor will be dosed immediately before the rats are fed. The CB₁ antagonist will be dosed 1h before the lipase inhibitor is administered.

As an example, the following daily dosing schedule is applicable for a given period of e.g. days, weeks or months:
The CB₁ antagonist of formula (I) as defined above, or a vehicle dose is administered (po) in the morning at ca. 07.45 to 08.00 h. The lipase inhibitor, e.g. in particular orlistat, or a vehicle dose is administered (po) ca. 08.45 to 09.00 h. After medication the rats are set to ad-libitum feed from 09.15 to 11.45h, followed by feed removal from about 11.45 to 14.45 h. Another dose of lipase inhibitor, e.g. in particular orlistat, or vehicle (Labrasol) dose is administered (po) in the afternoon at about 14.15 to 14.30h, followed by ad-libitum feed from 14.45 to 17.15 h. Thereafter, the rats are set to feed deprivation from 17.15 - 09.15 h.

The experimental protocol results will compare daily food intake and body weight gain as indicators for the effects of the combination treatment on obesity during the experimental phase. In addition to the before given parameters it may be desired to also collect faeces and to estimate fat digestibility. Eventually it may be also desired to perform a carcass analysis.

Furthermore, after finishing the experimental feeding and administering phase, biochemical parameters may be measured at slaughter of the rats.

For investigating the effects the total number of rats subject to the experimental protocol is divided into the following groups with approximately the same number of rats in each group:
1) Control group: The rats receive only vehicle according to the protocol to simulate administration (placebo group).
2) CB₁ group: The rats receive a CB₁ antagonist in a vehicle.
3) LI group: The rats receive as lipase inhibitor ("Ll") e.g. the compound orlistat in a vehicle.
4) CB₁+LI group (combination group): The rats receive a CB₁ antagonist in a vehicle and as lipase inhibitor ("LI") e.g. the compound orlistat in a vehicle.

The results of this protocol and the respective investigations show the beneficial suitability of the combination of a CB₁ antagonist and a lipase inhibitor in the treatment and / or prophylaxis of obesity.

## Claims

1. Use of a 4,5-dihydro-1 H-pyrazole, tautomer or salt thereof, of formula (I) which is a potent and selective antagonist of the cannabis CB₁-receptor 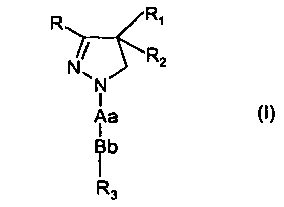 wherein
- R and R₁ are the same or different and represent phenyl, thienyl or pyridyl which groups may be substituted with 1, 2 or 3 substituents Y, which can be the same or different, from the group C₁₋₃-alkyl or alkoxy, hydroxy, halogen, trifluoromethyl, trifluoromethylthio, trifluoromethoxy, nitro, amino, mono- or dialkyl (C₁₋₂)-amino, mono- or dialkyl (C₁₋₂)-amido, (C₁₋₃)-alkyl sulfonyl, dimethylsulfamido, C₁₋₃-alkoxycarbonyl, carboxyl, trifluoromethylsulfonyl, cyano, carbamoyl, sulfamoyl and acetyl, or R and/or R₁ represent naphthyl,
- R₂ represents hydrogen, hydroxy, C₁₋₃-alkoxy, acetyloxy or propionyloxy,
- Aa represents one of the groups (i), (ii), (iii), (iv) or (v) 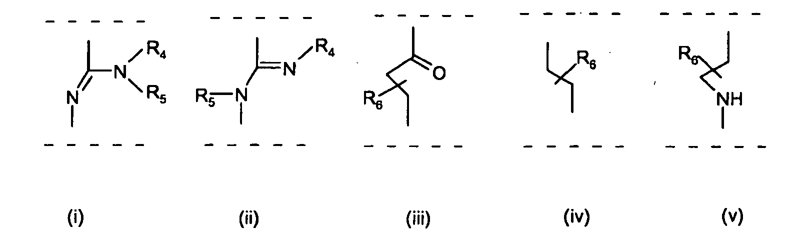 wherein
- R₄ and R₅ independently of each other represent hydrogen or C₁₋₈ branched or unbranched alkyl or C₃₋₈ cycloalkyl or R₄ represents acetamido or dimethylamino or 2,2,2-trifluoroethyl or phenyl or pyridyl with the proviso that R₅ represents hydrogen,
- R₆ represents hydrogen or C₁₋₃ unbranched alkyl;
- Bb represents sulfonyl or carbonyl,
- R₃ represents benzyl, phenyl, thienyl or pyridyl which may be substituted with 1, 2 or 3 substituents Y, which can be the same or different, or R₃ represents C₁₋₈ branched or unbranched alkyl or C₃₋₈ cycloalkyl, or R₃ represents naphthyl,
in combination with at least one lipase inhibiting compound selected from the group consisting of:
- orlistat
- 2-oxy-4H-3,1-benzoxazin-4-ones, preferably ATL-962, having the formula (II) or a pharmaceutically acceptable salt, ester or amide thereof: 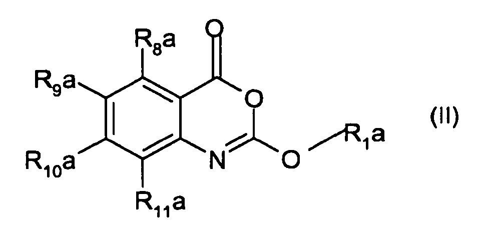
wherein:
R1a is
(i) a C10-30 branched or unbranched alkyl , optionally substituted by one or more independently of C3-6 cycloalkyl, C3-6 cycloalkenyl, aryl, heteroaryl, reduced heteroaryl, --C(O)R13, --CO2R13, --SOR13, -SO2R13, --NR13R14, --OR13, --SR13, --C(O)NR13R14, -NR14C(O)R13, halogen, cyano, and nitro and/or optionally interrupted by one or more oxygen atoms with the proviso that any hetero atom in R1 a must be separated from the exocyclic oxygen atom (or from any other heteroatom) by at least two carbon atoms;
(ii) C2-25 alkenyl, C2-25 alkynyl, C3-6 cycloalkenyl, aryl-C2-25 alkenyl, heteroaryl-C2-25 alkenyl, reduced heteroaryl, reduced heteroaryl-C1-25 alkyl or a substituted derivative of any of the foregoing groups wherein the substituents are one or more independently of C1-6 alkyl, halosubstituted C1-6 alkyl, aryl, aryl-C1-6 alkyl, heteroaryl, reduced heteroaryl, reduced heteroaryl-C1-6 alkyl, C1-6 alkoxy, aryl-C1-6 alkoxy, --C(O)R13, --CO2R13, --SOR13, --SO2R13, --NR13R14, --OR13, --SR13, --C(O)NR13R14, --NR14C(O)R13, halogen, cyano, and nitro, with the proviso that any hetero atom in R1a must be separated from the exocyclic oxygen atom (or from any other heteroatom) by at least two carbon atoms;
(iii) a C2-9 alkyl group interrupted by one or more oxygen atoms and optionally substituted by one or more independently of C3-6 cycloalkyl, C3-6 cycloalkenyl, aryl, heteroaryl, reduced heteroaryl, --C(O)R13, --CO2R13, --SOR13, --SO2R13, NR13R14, OR13, SR13, --C(O)NR13R14, --NR14C(O)R13, halogen, cyano and nitro with the proviso that any hetero atom in R1a must be separated from the exocyclic oxygen atom (or from any other heteroatom) by at least two carbon atoms; or
(iv) a C1-9 alkyl group substituted by a group selected from --C(O)R13, --CO2R13, SOR13, SO2R13, NR13R14, OR13, SR13, C(O)NR13R14, NR14C(O)R13; tetrahydronaphthyl, pyridyl, pyrrolyl, piperidinyl, halogen, cyano, nitro, bicyclic aryl, bicyclic heteroaryl, monocyclic or bicyclic reduced heteroaryl, monocyclic heteroaryl other than imidazolyl;
(v) a phenyl group substituted by a group selected from OR17, --COR13, --CO2R13, SOR13, SO2R13, CONR13R14, NR14C(O)R13 ; halosubstituted C1-6 alkyl, aryl, arylC1-6 alkyl, heteroaryl and heteroarylC1-6 alkyl; or
(vi) a bicyclic aryl, bicyclic heteroaryl, monocyclic or bicyclic reduced heteroaryl, or monocyclic heteroaryl group other than imidazolyl, optionally substituted by a group selected from OR17, --COR13, --CO2R13, SOR13, SO2R13, CONR13R14, NR14C(O)R13; halosubstituted C1-6 alkyl, aryl, arylC1-6 alkyl, heteroaryl and heteroarylC1-6 alkyl;
where R13 and R14 each independently represents hydrogen, C1-10 alkyl, C2-10 alkenyl, C2-10 alkynyl, C3-6 cycloalkyl, C3-6 cycloalkenyl, aryl, arylC1-10 alkyl, heteroaryl, heteroarylC1-10 alkyl, reduced heteroaryl or reduced heteroarylC1-10 alkyl, and R17 represents hydrogen or C2-10 alkenyl, C2-10 alkynyl, C3-6 cycloalkyl, C3-6 cycloalkenyl, aryl, arylC1-10 alkyl, heteroaryl, heteroarylC1-10 alkyl, reduced heteroaryl or reduced heteroarylC1-10 alkyl
and R8a, R9a, R10a and R11a are each independently hydrogen, halo, hydroxy, amino, nitro, cyano, thiol, C1-10 alkyl, C1-10 alkoxy, C1-10 cycloalkyl, C1-10 cycloalkoxy, C(O)R15, C(O)NR15R16, S(O)R15 or haloC1-10 alkyl;
where R15 and R16 each independently represent hydrogen or C1-10 alkyl with the proviso that when R8a, R9a, R10a, and R11a are H, R1a is not CH2CH2CI or C3 alkenyl
- lipstatin
- ebelactone B
- and panclicins,
in the manufacture of a medicament for the treatment and/or prophylaxis of obesity in juvenile patients and/or drug induced obesity in juvenile as well as adolescent patients.

2. Use of the compound having formula (I) according to claim 1, wherein R is the group 4-chlorophenyl, R₁ is phenyl, R₂ is hydrogen, Aa is the group (i) wherein R₄ is hydrogen and R₅ is methyl, Bb is sulfonyl, and R₃ represents 4-chlorophenyl, and salts thereof.

3. Use of the compound having formula (I) according to claim 1, wherein the compound is a levorotatory enantiomer.

4. Use of the compound having formula (I) according to anyone of the claims 1 to 3 wherein the at least one lipase inhibiting compound is selected from the group of: orlistat, panclicins, ATL-962 and lipstatin.

5. A 4,5-dihydro-1 H-pyrazole, tautomer or salt thereof, of formula (I) which is a potent and selective antagonist of the cannabis CB₁-receptor 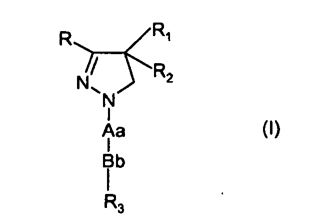 wherein the formula (I) is defined as described in Claim 1
in combination with at least one lipase inhibiting compound selected from the group consisting of:
- orlistat
- 2-oxy-4H-3,1-benzoxazin-4-ones, preferably ATL-962, having the formula (II) or a pharmaceutically acceptable salt, ester or amide thereof: 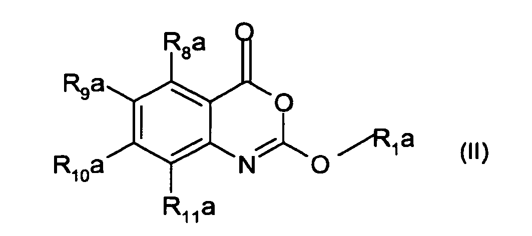
wherein: the formula (II) is defined as described in Claim 1
- lipstatin
- ebelactone B
- and panclicins,
for use in the treatment of obesity in juvenile patients and/or for use in the treatment of drug induced obesity in juvenile as well as adolescent patients.

6. Combination of compounds for use according to claim 5, **characterized in that** the treatment is directed to obesity in juvenile patients.

7. Combination of compounds for use according to claim 5, **characterized in that** the treatment is directed to drug induced obesity in juvenile or adolescent patients.

8. Combination of compounds for use according to any of claims 5 to 7, **characterized in that** the 4,5-dihydro-1H-pyrazole is to be used in combination with the lipase inhibiting compound by simultaneous, separate or step-wise administration route.

9. Combination of compounds for use according to claim 5, wherein R is the group 4-chlorophenyl, R₁ is phenyl, R₂ is hydrogen, Aa is the group (i) wherein R₄ is hydrogen and R₅ is methyl, Bb is sulfonyl, and R₃ represents 4-chlorophenyl, and salts thereof.

10. Combination of compounds for use according to claim 5, wherein the compound having formula (I) is a levorotatory enantiomer.

11. Combination of compounds for use according to claim 5 to 10, wherein the at least one lipase inhibiting compound is selected from the group of: orlistat, panclicins, ATL-962 and lipstatin.

12. Pharmaceutical product containing as a medicament a 4,5-dihydro-1H-pyrazole, tautomer or salt thereof, of formula (I) which is a potent and selective antagonist of the cannabis CB₁-receptor 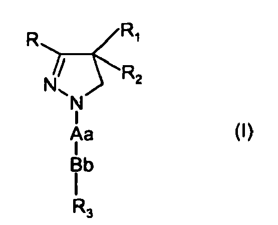 wherein the formula (I) is defined as described in Claim 1
as a combination preparation with a lipase inhibiting compound selected from the group consisting of:
- orlistat
- 2-oxy-4H-3,1-benzoxazin-4-ones, preferably ATL-962, having the formula (II) or a pharmaceutically acceptable salt, ester or amide thereof: 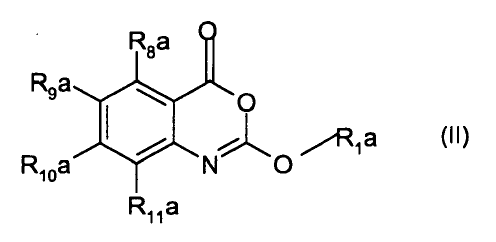
wherein the formula (II) is defined as described in Claim 1
- lipstatin
- ebelactone B
- and panclicins,
for use by simultaneous, separate or step-wise administration in the treatment and/or prophylaxis of obesity in juvenile patients and/or drug induced obesity in juvenile as well as adolescent patients.

## Patentansprüche

1. Verwendung eines 4,5-Dihydro-1H-pyrazols oder eines Tautomers oder Salzes davon der Formel (I), bei dem es sich um einen wirksamen und selektiven Antagonisten des Cannabis-CB₁-Rezeptors handelt, 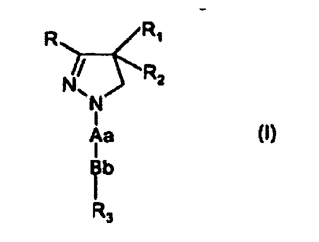 wobei
- R und R₁ gleich oder verschieden sind und für Phenyl, Thienyl oder Pyridyl stehen, wobei diese Gruppen durch 1, 2 oder 3 gleiche oder verschiedene Substituenten Y aus der Gruppe C₁₋₃-Alkyl oder Alkoxy, Hydroxy, Halogen, Trifluormethyl, Trifluormethylthio, Trifluormethoxy, Nitro, Amino, Mono- oder Dialkyl-(C₁₋₂)-amino, Mono- oder Dialkyl-(C₁₋₂)-amido, (C₁₋₃) -Alkylsulfonyl, Dimethylsulfamido, C₁₋₃-Alkoxycarbonyl, Carboxyl, Trifluormethylsulfonyl, Cyano, Carbamoyl, Sulfamoyl und Acetyl substituiert sein können, oder R und/oder R₁ für Naphthyl stehen,
- R₂ für Wasserstoff, Hydroxy, C₁₋₃-Alkoxy, Acetyloxy oder Propionyloxy steht,
- Aa für eine der Gruppen (i), (ii), (iii), (iv) oder (v) steht, 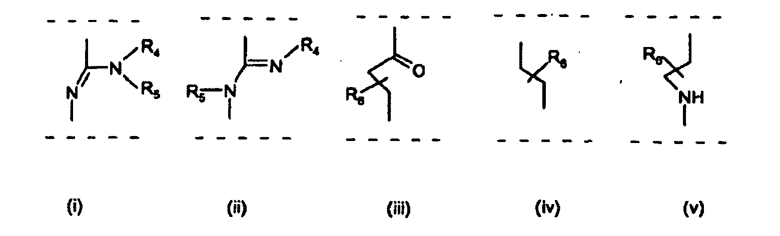 wobei
- R₄ und R₅ unabhängig voneinander für Wasserstoff oder verzweigtes oder geradkettiges C₁₋₈-Alkyl oder C₃₋₈-Cycloalkyl stehen oder R₄ für Acetamido oder Dimethylamino oder 2,2,2-Trifluorethyl oder Phenyl oder Pyridyl steht, mit der Maßgabe, daß R₅ für Wasserstoff steht,
- R₆ für Wasserstoff oder geradkettiges C₁₋₃-Alkyl steht;
- Bb für Sulfonyl oder Carbonyl steht,
- R₃ für Benzyl, Phenyl, Thienyl oder Pyridyl steht, das durch 1, 2 oder 3 gleiche oder verschiedene Substituenten Y substituiert sein kann, oder R₃ für verzweigtes oder geradkettiges C₁₋₈-Alkyl oder C₃₋₈-Cycloalkyl steht, oder R₃ für Naphthyl steht,
in Kombination mit wenigstens einer lipasehemmenden Verbindung ausgewählt aus der Gruppe bestehend aus:
- Orlistat
- 2-Oxy-4H-3,1-benzoxazin-4-one, vorzugsweise ATL-962, vorzugsweise der Formel (II) oder einem pharmazeutisch unbedenklichen Salz, Ester oder Amid davon: 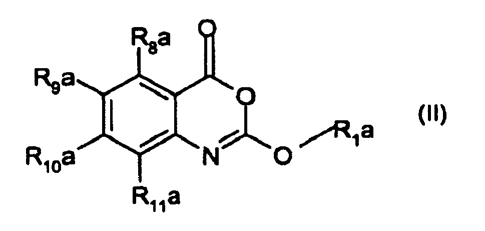
wobei:
R1a für
(i) verzweigtes oder geradkettiges C10-30-Alkyl, gegebenenfalls substituiert durch ein oder mehrere Substituenten unabhängig voneinander ausgewählt aus C3-6-Cycloalkyl, C3-6-Cycloalkenyl, Aryl, Heteroaryl, reduziertem Heteroaryl, --C(O)R13, --CO2R13, --SOR13, --SO2R13, --NR13R14, --OR13, --SR13, --C(O)NR13R14, --NR14C(O)R13, Halogen, Cyano und Nitro und/oder gegebenenfalls unterbrochen durch ein oder mehrere Sauerstoffatome, mit der Maßgabe, daß alle Heteroatome in R1a von dem exocyclischen Sauerstoffatom (oder von einem anderen Heteroatom) durch wenigstens zwei Kohlenstoffatome getrennt sein müssen, steht;
(ii) C2-25-Alkenyl, C2-25-Alkynyl, C3-6-Cycloalkenyl, Aryl-C2-25-alkenyl, Heteroaryl-C2-25-alkenyl, reduziertes Heteroaryl, reduziertes Heteroaryl-C1-25-alkyl oder ein substituiertes Derivat einer der obigen Gruppen steht, wobei es sich bei den Substituenten um ein oder mehrere unabhängig voneinander aus C1-6-Alkyl, halogensubstituiertem C1-6-Alkyl, Aryl, Aryl-C1-6-alkyl, Heteroaryl, reduziertem Heteroaryl, reduziertem Heteroaryl-C1-6-alkyl, C1-6-Alkoxy, Aryl-C1-6-alkoxy, --C(O)R13, --CO2R13, --SOR13, --SO2R13, --NR13R14, --OR13, --SR13, --C(O)NR13R14, --NR14C(O)R13, Halogen, Cyano und Nitro ausgewählte Substituenten handelt, mit der Maßgabe, daß alle Heteroatome in R1a von dem exocyclischen Sauerstoffatom (oder von einem anderen Heteroatom) durch wenigstens zwei Kohlenstoffatome getrennt sein müssen, steht;
(iii) eine C2-9-Alkylgruppe, unterbrochen durch ein oder mehrere Sauerstoffatome und gegebenenfalls substituiert durch ein oder mehrere unabhängig voneinander aus C3-6-Cycloalkyl, C3-6-Cycloalkenyl, Aryl, Heteroaryl, reduziertem Heteroaryl, --C(O)R13, --CO2R13, --SOR13, --SO2R13, NR13R14, OR13, SR13, --C(O)NR13R14, --NR14C(O)R13, Halogen, Cyano und Nitro ausgewählte Substituenten, mit der Maßgabe, daß alle Heteroatome in R1a von dem exocyclischen Sauerstoffatom (oder von einem anderen Heteroatom) durch wenigstens zwei Kohlenstoffatome getrennt sein müssen, steht; oder
(iv) eine C1-9-Alkylgruppe substituiert durch eine Gruppe ausgewählt aus --C(O)R13, --CO2R13, SOR13, SO2R13, NR13R14, OR13, SR13, C(O)NR13R14, NR14C(O)R13; Tetrahydronaphthyl, Pyridyl, Pyrrolyl, Piperidinyl, Halogen, Cyano, Nitro, bicyclischem Aryl, bicyclischem Heteroaryl, monocyclischem oder bicyclischem reduziertem Heteroaryl, monocyclischem Heteroaryl mit Ausnahme von Imidazolyl steht;
(v) eine Phenylgruppe substituiert durch eine Gruppe ausgewählt aus OR17, --COR13, --CO2R13, SOR13, SO2R13, CONR13R14, NR14C(O)R13; halogensubstituiertem C1-6-Alkyl, Aryl, Aryl-C1-6-alkyl, Heteroaryl und Heteroaryl-C1-6-alkyl steht; oder
(vi) bicyclisches Aryl, bicyclisches Heteroaryl, monocyclisches oder bicyclisches reduziertes Heteroaryl oder eine monocyclische Heteroarylgruppe mit Ausnahme von Imidazolyl, gegebenenfalls substituiert durch eine Gruppe ausgewählt aus OR17, --COR13, --CO2R13, SOR13, SO2R13, CONR13R14, NR14C(O)R13; halogensubstituiertem C1-6-Alkyl, Aryl, Aryl-C1-6-alkyl, Heteroaryl und Heteroaryl-C1-6-alkyl, steht;
wobei R13 und R14 jeweils unabhängig voneinander für Wasserstoff, C1-10-Alkyl, C2-10-Alkenyl, C2-10-Alkynyl, C3-6-Cycloalkyl, C3-6-Cycloalkenyl, Aryl, Aryl-C1-10-alkyl, Heteroaryl, Heteroaryl-C1-10-alkyl, reduziertes Heteroaryl oder reduziertes Heteroaryl-C1-10-alkyl stehen und R17 für Wasserstoff oder C2-10-Alkenyl, C2-10-Alkynyl, C3-6-Cycloalkyl, C3-6-Cycloalkenyl, Aryl, Aryl-C1-10-alkyl, Heteroaryl, Heteroaryl-C1-10-alkyl, reduziertes Heteroaryl oder reduziertes Heteroaryl-C1-10-alkyl steht,
und R8a, R9a, R10a und R11a jeweils unabhängig voneinander für Wasserstoff, Halogen, Hydroxy, Amino, Nitro, Cyano, Thiol, C1-10-Alkyl, C1-10-Alkoxy, C1-10-Cycloalkyl, C1-10-Cycloalkoxy, C(O)R15, C(O)NR15R16, S(O)R15 oder Halogen-C1-10-alkyl stehen;
wobei R15 und R16 jeweils unabhängig voneinander für Wasserstoff oder C1-10-Alkyl stehen, mit der Maßgabe, daß, wenn R8a, R9a, R10a und R11a für H stehen, R1a nicht für CH2CH2Cl oder C3-Alkenyl steht
- Lipstatin
- Ebelacton B
- und Panclicinen,
bei der Herstellung eines Medikaments zur Behandlung und/oder Prophylaxe von Obesitas in jugendlichen Patienten und/oder arzneimittelinduzierter Obesitas in jugendlichen sowie heranwachsenden Patienten.

2. Verwendung der Verbindung der Formel (I) nach Anspruch 1, wobei R für die Gruppe 4-Chlorphenyl steht, R₁ für Phenyl steht, R₂ für Wasserstoff steht, Aa für die Gruppe (i) steht, wobei R₄ für Wasserstoff steht und R₅ für Methyl steht, Bb für Sulfonyl steht und R₃ für 4-Chlorphenyl steht, und Salzen davon.

3. Verwendung der Verbindung der Formel (I) nach Anspruch 1, wobei es sich bei der Verbindung um das linksdrehende Enantionmer handelt.

4. Verwendung der Verbindung der Formel (I) nach einem der Ansprüche 1 bis 3, wobei die wenigstens eine lipasehemmende Verbindung aus der aus Orlistat, Panclicinen, ATL-962 und Lipstatin bestehenden Gruppe ausgewählt ist.

5. 4,5-Dihydro-1H-pyrazole und deren Tautomere und Salze der Formel (I), bei denen es sich um wirksame und selektive Antagonisten des Cannabis-CB₁-Rezeptors handelt, 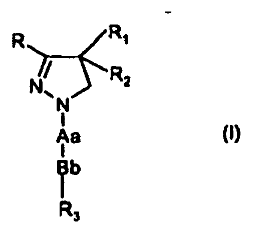 wobei die Formel (I) wie in Anspruch 1 beschrieben definiert ist,
in Kombination mit wenigstens einer lipasehemmenden Verbindung ausgewählt aus der Gruppe bestehend aus :
- Orlistat
- 2-Oxy-4H-3,1-benzoxazin-4-onen, vorzugsweise ATL-962, mit der Formel (II) oder ein pharmazeutisch unbedenkliches Salz, ein pharmazeutisch unbedenklicher Ester oder ein pharmazeutisch unbedenkliches Amid davon: 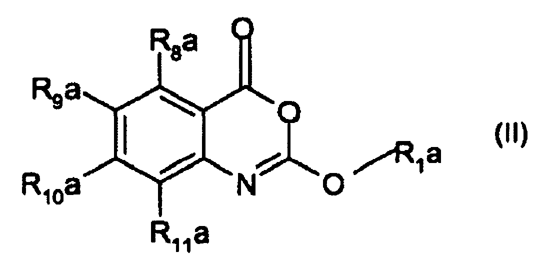
wobei:
die Formel (II) wie in Anspruch 1 beschrieben definiert ist
- Lipstatin
- Ebelacton B
- und Panclicinen,
zur Verwendung bei der Behandlung von Obesitas in jugendlichen Patienten und/oder zur Verwendung bei der Behandlung arzneimittelinduzierter Obesitas in jugendlichen sowie heranwachsenden Patienten.

6. Kombination von Verbindungen zur Verwendung gemäß Anspruch 5, **dadurch gekennzeichnet, daß** die Behandlung auf Obesitas in jugendlichen Patienten zielt.

7. Kombination von Verbindungen zur Verwendung gemäß Anspruch 5, **dadurch gekennzeichnet, daß** die Behandlung auf arzneimittelinduzierte Obesitas in Jugendlichen oder heranwachsenden Patienten zielt.

8. Kombination von Verbindungen zur Verwendung gemäß einem der Ansprüche 5 bis 7, **dadurch gekennzeichnet, daß** das 4,5-Dihydro-1H-pyrazol durch gleichzeitige, getrennte oder schrittweise Verabreichung in Kombination mit der lipasehemmenden Verbindung anzuwenden ist.

9. Kombination von Verbindungen zur Verwendung gemäß Anspruch 5, wobei R für die Gruppe 4-Chlorphenyl steht, R₁ für Phenyl steht, R₂ für Wasserstoff steht, Aa für die Gruppe (i) steht, wobei R₄ für Wasserstoff steht und R₅ für Methyl steht, Bb für Sulfonyl steht und R₃ für 4-Chlorphenyl steht, und Salzen davon.

10. Kombination von Verbindungen zur Verwendung gemäß Anspruch 5, wobei es sich bei der Verbindung der Formel (I) um ein linksdrehendes Enantiomer handelt.

11. Kombination von Verbindungen zur Verwendung gemäß einem der Ansprüche 5 bis 10, wobei die wenigstens eine lipasehemmende Verbindung aus der aus Orlistat, Panclicinen, ATL-962 und Lipstatin bestehenden Gruppe ausgewählt ist.

12. Pharmazeutisches Produkt, enthaltend als Medikament ein 4,5-Dihydro-1H-pyrazol, ein Tautomer oder ein Salz davon der Formel (I), bei dem es sich um einen wirksamen und selektiven Antagonisten des Cannabis-CB₁-Rezeptors handelt, 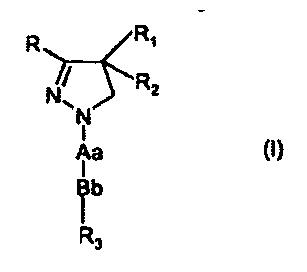 wobei die Formel (I) wie in Anspruch 1 beschrieben definiert ist,
als Kombinationspräparat mit einer lipasehemmenden Verbindung ausgewählt aus der Gruppe bestehend aus:
- Orlistat
- 2-Oxy-4H-3,1-benzoxazin-4-onen, vorzugsweise ATL-962, mit der Formel (II) oder einem pharmazeutisch unbedenklichen Salz, Ester oder Amid davon: 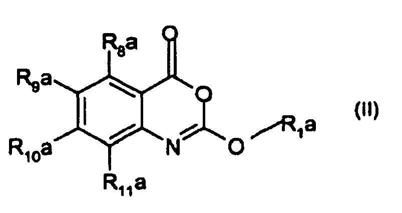
wobei die Formel (II) wie in Anspruch 1 beschrieben definiert ist
- Lipstatin
- Ebelacton B
- und Panclicinen,
zur Verwendung durch gleichzeitige, getrennte oder schrittweise Verabreichung bei der Behandlung und/oder Prophylaxe von Obesitas in jugendlichen Patienten und/oder arzneimittelinduzierter Obesitas in jugendlichen sowie heranwachsenden Patienten.

## Revendications

1. Utilisation d'un 4,5-dihydro-1H-pyrazole, d'un tautomère ou d'un sel de celui-ci, de formule (I) qui est un antagoniste puissant et sélectif du récepteur au cannabis CB₁ 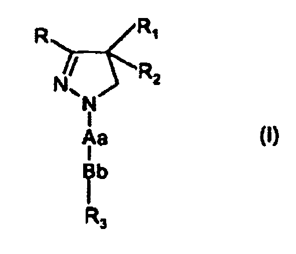 dans laquelle
- R et R₁ sont identiques ou différents et représentent phényle, thiényle ou pyridyle, lesquels groupements peuvent être substitués par 1, 2 ou 3 substituants Y, qui peuvent être identiques ou différents, du groupe C₁₋₃-alkyle ou alcoxy, hydroxy, halogène, trifluorométhyle, trifluorométhylthio, trifluorométhoxy, nitro, amino, mono- ou dialkyl-(C₁₋₂)-amino, mono- ou dialkyl-(C₁₋₂) - amido, (C₁₋₃)-alkylsulfonyle, diméthylsulfamido, C₁₋₃-alcoxycarbonyle, carboxy, trifluorométhylsulfonyle, cyano, carbamoyle, sulfamoyle et acétyle,
ou R et/ou R₁ représentent naphtyle,
- R₂ représente hydrogène, hydroxy, C₁₋₃-alcoxy, acétyloxy ou propionyloxy,
- Aa représente l'un des groupements (i), (ii), (iii), (iv) ou (v) 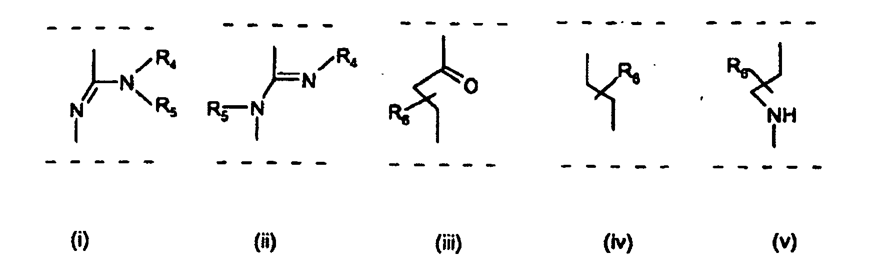 dans lesquels
- R₄ et R₅, indépendamment l'un de l'autre, représentent hydrogène ou C₁₋₈-alkyle ramifié ou non ramifié ou C₃₋₈-cycloalkyle ou R₄ représente acétamido ou diméthylamino ou 2,2,2-trifluoroéthyle ou phényle ou pyridyle, à condition que R₅ représente hydrogène,
- R₆ représente hydrogène ou C₁₋₃-alkyle non ramifié ;
- Bb représente sulfonyle ou carbonyle,
- R₃ représente benzyle, phényle, thiényle ou pyridyle qui peuvent être substitués par 1, 2 ou 3 substituants Y, qui peuvent être identiques ou différents, ou R₃ représente C₁-₈-alkyle ramifié ou non ramifié ou C₃₋₈-cycloalkyle, ou R₃ représente naphtyle,
en combinaison avec au moins un composé inhibiteur de lipase choisi dans le groupe constitué de :
- orlistat
- 2-oxy-4H-3,1-benzoxazin-4-ones, de préférence ATL-962, ayant la formule (II) ou un sel, un ester ou un amide pharmaceutiquement acceptable de celles-ci : 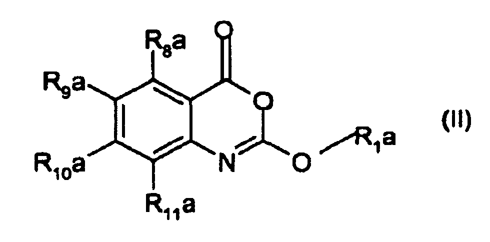
dans laquelle :
R1a est
(i) un C10-30-alkyle ramifié ou non ramifié, éventuellement substitué par un ou plusieurs indépendamment parmi C3-6-cycloalkyle, C3-6-cycloalcényle, aryle, hétéroaryle, hétéroaryle réduit, --C(O)R13, --CO2R13, --SOR13, --SO2R13, --NR13R14, --OR13, --SR13, --C(O)NR13R14, --NR14C(O)R13, halogène, cyano, et nitro et/ou éventuellement interrompu par un ou plusieurs atomes d'oxygène à condition que tout hétéroatome dans R1a soit séparé de l'atome d'oxygène exocyclique (ou de tout autre hétéroatome) par au moins deux atomes de carbone ;
(ii) C2-25-alcényle, C2-25-alcynyle, C3-6-cycloalcényle, aryl-C2-25-alcényle, hétéroaryl-C2-25-alcényle, hétéroaryle réduit, hétéroaryl-C1-25-alkyle réduit ou un dérivé substitué de l'un quelconque des groupements précédents où les substituants sont un ou plusieurs indépendamment parmi C1-6-alkyle, C1-6-alkyle halogéno-substitué, aryle, aryl-C1-6-alkyle, hétéroaryle, hétéroaryle réduit, hétéroaryl-C1-6-alkyle réduit, C1-6-alcoxy, aryl-C1-6-alcoxy, --C(O)R13, --CO2R13, --SOR13, --SO2R13, --NR13R14, --OR13, --SR13, --C(O)NR13R14, --NR14C(O)R13, halogène, cyano, et nitro, à condition que tout hétéroatome dans R1a soit séparé de l'atome d'oxygène exocyclique (ou de tout autre hétéroatome) par au moins deux atomes de carbone ;
(iii) un groupement C2-9-alkyle interrompu par un ou plusieurs atomes d'oxygène et éventuellement substitué par un ou plusieurs indépendamment parmi C3-6-cycloalkyle, C3-6-cycloalcényle, aryle, hétéroaryle, hétéroaryle réduit, --C(O)R13, --CO2R13, --SOR13, --SO2R13, NR13R14, OR13, SR13, --C(O)NR13R14, --NR14C(O)R13, halogène, cyano et nitro à condition que tout hétéroatome dans R1a soit séparé de l'atome d'oxygène exocyclique (ou de tout autre hétéroatome) par au moins deux atomes de carbone ; ou
(iv) un groupement C1-9-alkyle substitué par un groupement choisi parmi --C(O)R13, --CO2R13, SOR13, SO2R13, NR13R14, OR13, SR13, C(O)NR13R14, NR14C(O)R13; tétrahydronaphtyle, pyridyle, pyrrolyle, pipéridinyle, halogène, cyano, nitro, aryle bicyclique, hétéroaryle bicyclique, hétéroaryle monocyclique ou bicyclique réduit, hétéroaryle monocyclique autre qu'imidazolyle ;
(v) un groupement phényle substitué par un groupement choisi parmi OR17, --COR13, --CO2R13, SOR13, SO2R13, CONR13R14, NR14C(O)R13 ; C1-6-alkyle halogéno-substitué, aryle, aryl-C1-6-alkyle, hétéroaryle et hétéroaryl-C1-6-alkyle ; ou
(vi) un groupement aryle bicyclique, hétéroaryle bicyclique, hétéroaryle monocyclique ou bicyclique réduit, ou hétéroaryle monocyclique autre qu'imidazolyle, éventuellement substitué par un groupement choisi parmi OR17, --COR13, --CO2R13, SOR13, SO2R13, CONR13R14, NR14C(O)R13; C1-6-alkyle halogéno-substitué, aryle, aryl-C1-6-alkyle, hétéroaryle et hétéroaryl-C1-6-alkyle ;
où R13 et R14 représentent chacun indépendamment hydrogène, C1-10-alkyle, C2-10-alcényle, C2-10-alcynyle, C3-6-cycloalkyle, C3-6-cycloalcényle, aryle, aryl-C1-10-alkyle, hétéroaryle, hétéroaryl-C1-10-alkyle, hétéroaryle réduit ou hétéroaryl-C1-10-alkyle réduit, et R17 représente hydrogène ou C2-10-alcényle, C2-10-alcynyle, C3-6-cycloalkyle, C3-6-cycloalcényle, aryle, aryl-C1-10-alkyle, hétéroaryle, hétéroaryl-C1-10-alkyle, hétéroaryle réduit ou hétéroaryl-C1-10-alkyle réduit
et R8a, R9a, R10a et R11a sont chacun indépendamment hydrogène, halogéno, hydroxy, amino, nitro, cyano, thiol, C1-10-alkyle, C1-10-alcoxy, C1-10-cycloalkyle, C1-10-cycloalcoxy, C(O)R15, C(O)NR15R16, S(O)R15 ou halogéno-C1-10-alkyle ;
où R15 et R16 représentent chacun indépendamment hydrogène ou C1-10-alkyle à condition que lorsque R8a, R9a, R10a et R11a sont H, R1a ne soit pas CH2CH2Cl ou C3-alcényle
- lipstatine
- ébélactone B
- et panclicines,
dans la fabrication d'un médicament destiné au traitement et/ou à la prophylaxie de l'obésité chez les patients juvéniles et/ou de l'obésité médicamenteuse chez les patients juvéniles et adolescents.

2. Utilisation du composé ayant la formule (I) selon la revendication 1, **caractérisée en ce que** R est le groupement 4-chlorophényle, R₁ est phényle, R₂ est hydrogène, Aa est le groupement (i) où R₄ est hydrogène et R₅ est méthyle, Bb est sulfonyle, et R₃ représente 4-chlorophényle, et des sels de celui-ci.

3. Utilisation du composé ayant la formule (I) selon la revendication 1, **caractérisée en ce que** le composé est un énantiomère lévogyre.

4. Utilisation du composé ayant la formule (I) selon l'une quelconque des revendications 1 à 3, **caractérisée en ce qu'**au moins un composé inhibiteur de lipase est choisi dans le groupe de : orlistat, panclicines, ATL-962 et lipstatine.

5. 4,5-Dihydro-1H-pyrazole, un tautomère ou un sel de celui-ci, de formule (I), qui est un antagoniste puissant et sélectif du récepteur au cannabis CB₁ 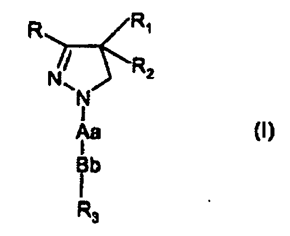 où la formule (I) est définie comme décrit dans la revendication 1
en combinaison avec au moins un composé inhibiteur de lipase choisi dans le groupe constitué de :
- orlistat
- 2-oxy-4H-3,1-benzoxazin-4-ones, de préférence ATL-962, ayant la formule (II) ou un sel, ester
ou amide pharmaceutiquement acceptable de celles-ci : 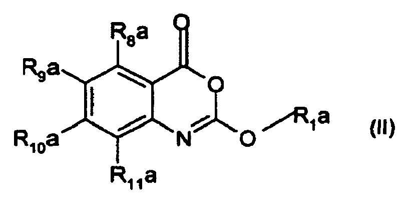 où : la formule (II) est définie comme décrit dans la revendication 1
- lipstatine
- ébélactone B
- et panclicines,
pour une utilisation dans le traitement de l'obésité chez les patients juvéniles et/ou pour une utilisation dans le traitement de l'obésité médicamenteuse chez les patients juvéniles et adolescents.

6. Combinaison de composés destinée à être utilisée selon la revendication 5, **caractérisée en ce que** le traitement vise l'obésité chez les patients juvéniles.

7. Combinaison de composés destinée à être utilisée selon la revendication 5, **caractérisée en ce que** le traitement vise l'obésité médicamenteuse chez les patients juvéniles ou adolescents.

8. Combinaison de composés destinée à être utilisée selon l'une quelconque des revendications 5 à 7, **caractérisée en ce que** le 4,5-dihydro-1H-pyrazole est utilisé en combinaison avec le composé inhibiteur de lipase par administration simultanée, séparée ou par étape.

9. Combinaison de composés destinée à être utilisée selon la revendication 5, **caractérisée en ce que** R est le groupement 4-chlorophényle, R₁ est phényle, R₂ est hydrogène, Aa est le groupement (i) où R₄ est hydrogène et R₅ est méthyle, Bb est sulfonyle, et R₃ représente 4-chlorophényle, et des sels de ceux-ci.

10. Combinaison de composés destinée à être utilisée selon la revendication 5, **caractérisée en ce que** le composé ayant la formule (I) est un énantiomère lévogyre.

11. Combinaison de composés destinée à être utilisée selon les revendications 5 à 10, **caractérisée en ce qu'**au moins un composé inhibiteur de lipase est choisi dans le groupe de : orlistat, panclicines, ATL-962 et lipstatine.

12. Produit pharmaceutique contenant, à titre de médicament, un 4,5-dihydro-1H-pyrazole, un tautomère ou un sel de celui-ci, de formule (I) qui est un antagoniste puissant et sélectif du récepteur au cannabis CB₁ 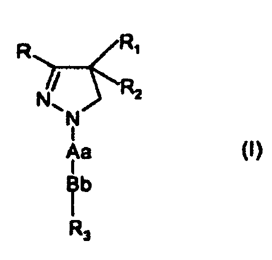 où la formule (I) est définie comme décrit dans la revendication 1
en tant que préparation combinée, avec un composé inhibiteur de lipase choisi dans le groupe constitué de :
- orlistat
- 2-oxy-4H-3,1-benzoxazin-4-ones, de préférence ATL-962, ayant la formule (II) ou un sel, ester ou amide pharmaceutiquement acceptable de celles-ci : 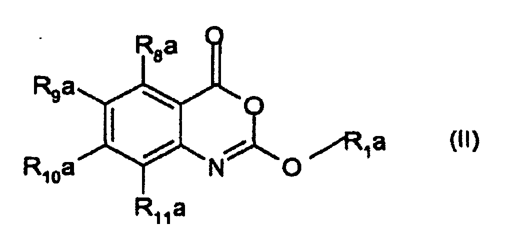
où la formule (II) est définie comme décrit dans la revendication 1
- lipstatine
- ébélactone B
- et panclicines,
pour une utilisation par administration simultanée, séparée ou par étape dans le traitement et/ou la prophylaxie de l'obésité chez les patients juvéniles et/ou de l'obésité médicamenteuse chez les patients juvéniles et adolescents.
